(19) Europäisches Patentamt / European Patent Office / Office européen des brevets

(11) **EP 3 915 474 A1**

(12) **EUROPEAN PATENT APPLICATION**
published in accordance with Art. 153(4) EPC

(43) Date of publication:
**01.12.2021 Bulletin 2021/48**

(21) Application number: **19912016.3**

(22) Date of filing: **24.01.2019**

(51) Int Cl.:
**A61B 5/11** *(2006.01)*

(86) International application number:
**PCT/JP2019/002212**

(87) International publication number:
**WO 2020/152817 (30.07.2020 Gazette 2020/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **FUJITSU LIMITED**
**Kanagawa 211-8588 (JP)**

(72) Inventors:
• **OYA, Takuro**
  **Kawasaki-shi, Kanagawa 211-8588 (JP)**
• **HOTTA, Shinji**
  **Kawasaki-shi, Kanagawa 211-8588 (JP)**
• **MAEDA, Kazuho**
  **Kawasaki-shi, Kanagawa 211-8588 (JP)**
• **AKI, Michihiko**
  **Kawasaki-shi, Kanagawa 211-8588 (JP)**

(74) Representative: **Hoffmann Eitle**
**Patent- und Rechtsanwälte PartmbB**
**Arabellastraße 30**
**81925 München (DE)**

(54) **INFORMATION PROCESSING PROGRAM, INFORMATION PROCESSING METHOD, AND INFORMATION PROCESSING SYSTEM**

(57) To calculate an index for accurately analyzing a circumduction gait, a server acquires data of change in an angular velocity over time of an ankle during walking detected by a sensing device attached to the ankle of a patient, and specifies a time of a swing phase based on change in an angular velocity over time around an X-axis. Furthermore, the server specifies a time from a beginning of the swing phase to a time when the angular velocity around the X-axis becomes maximum in the swing phase as a time of interest, and calculates, based on a width W of an angular velocity around a Z-axis within the time of interest, an index used to determine whether or not the patient is performing a circumduction gait.

FIG. 8

## Description

[Field]

The present invention relates to an information processing program, an information processing method, and an information processing system.

[Background Art]

For example, a patient who has collapsed from a stroke or the like may have a gait disturbance. In such a patient, to compensate for abnormality in a desired motion, another part may perform a movement (compensatory movement) to achieve the desired motion instead. Patients with stroke or the like have symptoms such as arm flexion and inversion. Thus, when such patients walk, they often perform a movement of rotating a leg (circumduction gait) as the compensatory movement.

Conventionally, a device that prevents a circumduction gait and a device that measures a circumduction gait are known (refer to, for example, Patent Documents 1 and 2).

[Citation List]

[Patent Literature]

[PTL 1] Japanese Laid-open Patent Publication No. 2004-195109.
[PTL 2] Japanese Laid-open Patent Publication No. 2016-43092

[Summary]

[Technical Problem]

However, with conventional technology, although it is possible to measure a swing of a leg in a right and left direction, it is not possible to measure at which stage of a walking cycle the swing is performed. Thus, it is not possible to perform accurate gait analysis from measurement results. Therefore, it is difficult to use such measurement results for follow-up, treatment, and the like of patients in an actual clinical site.

In one aspect, an object of the present invention is to provide an information processing program, an information processing method, and an information processing system that are capable of calculating an index for accurately analyzing a circumduction gait.

[Solution to Problem]

In one aspect, an information processing program is a program that causes a computer to execute processing of: acquiring information regarding an angular velocity of an ankle during walking detected by a sensor attached to the ankle of a person; detecting, among pieces of the acquired information regarding the angular velocity, a swing phase, which is a time when a foot is off a ground, on the basis of change in an angular velocity over time around a first axis that extends in a right and left direction of the person; specifying, as a time of interest, a predetermined time from a beginning of the swing phase; and calculating, among the pieces of the acquired information regarding the angular velocity, an index used to determine whether or not the person is performing a circumduction gait on the basis of change in an angular velocity over time around a second axis that extends in a vertical direction within the time of interest.

[Advantageous Effects of Invention]

It is possible to calculate an index for accurately analyzing a circumduction gait.

[Brief Description of Drawings]

[FIG. 1] FIG. 1 is a diagram schematically illustrating a configuration of an information processing system according to an embodiment.
[FIG. 2] FIG. 2A is a diagram illustrating a hardware configuration of a physical therapist terminal and a doctor terminal, and FIG. 2B is a diagram illustrating a hardware configuration of a server.
[FIG. 3] FIG. 3 is a diagram for describing a circumduction gait.
[FIG. 4] FIG. 4 is a diagram for describing a Rancho Los Amigos method.
[FIG. 5] FIG. 5 is a functional block diagram of the server.
[FIG. 6] FIG. 6 is a diagram illustrating an X-axis and a Z-axis set in a sensing device.
[FIG. 7] FIG. 7A is a diagram illustrating data of change in an angular velocity over time around the X-axis, and FIG. 7B is a diagram illustrating data of change in an angular velocity over time around the Z-axis.
[FIG. 8] FIG. 8 is a diagram for describing a method of calculating a feature amount.
[FIG. 9] FIG. 9 is a flowchart illustrating preprocessing by the sensing device.
[FIG. 10] FIG. 10 is a flowchart illustrating data analysis processing by the server.
[FIG. 11] FIGs. 11A and 11B are diagrams for describing the processing in FIG. 10.
[FIG. 12] FIG. 12A is a graph illustrating change in an output feature amount of a subject A, and FIG. 12B is a graph illustrating change in an output feature amount of a subject B.
[FIG. 13] FIG. 13 is a functional block diagram of a server according to a modification.

[FIG. 14] FIG. 14 is a diagram for describing processing of the server according to the modification.
[FIG. 15] FIG. 15 is a diagram for describing a measurement principle of a lateral movement amount according to the modification.

[Description of Embodiments]

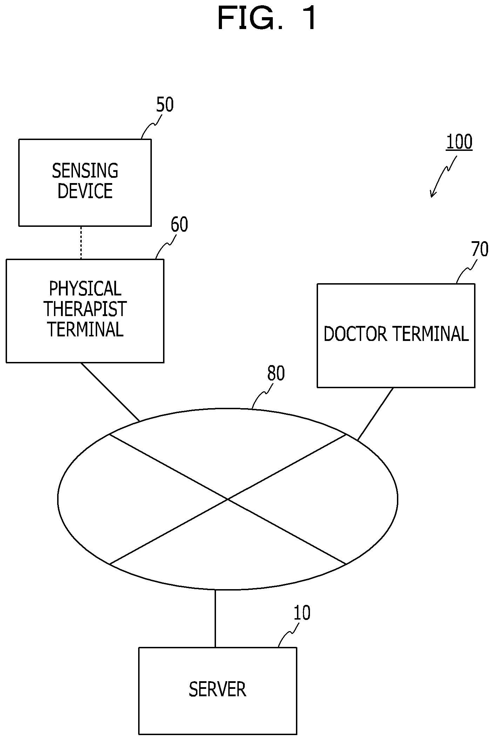

Hereinafter, an embodiment of an information processing system will be described in detail on the basis of FIGs. 1 to 12B. FIG. 1 schematically illustrates a configuration of an information processing system 100 according to an embodiment.

As illustrated in FIG. 1, the information processing system 100 includes a sensing device 50 as a sensor, a physical therapist terminal 60, a doctor terminal 70, and a server 10. The server 10, the physical therapist terminal 60, and the doctor terminal 70 are connected to a network 80 such as the Internet.

The sensing device 50 includes an angular velocity sensor, a control unit that controls detection of the angular velocity sensor, and a memory that stores a detection result of the angular velocity sensor. The sensing device 50 is provided on both ankles of a patient when the patient performs a walk test, which is one of motor function tests, under instructions of a physical therapist, and detects change in an angular velocity over time. It is assumed that the sensing device 50 is also provided with input buttons for inputting start and end of measurement.

The physical therapist terminal 60 is a terminal used by a physical therapist, such as a personal computer (PC) or a tablet-type terminal. The physical therapist terminal 60 is connected to the sensing device 50 to acquire a detection result (data indicating change in an angular velocity over time) of the sensing device 50. Furthermore, the physical therapist terminal 60 transmits the acquired data indicating change in an angular velocity over time to the server 10 via the network 80.

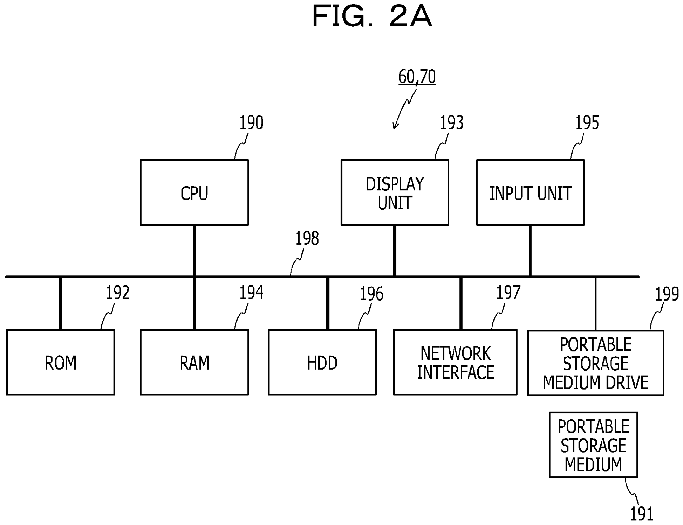

Here, FIG. 2A illustrates a hardware configuration of the physical therapist terminal 60. As illustrated in FIG. 2A, the physical therapist terminal 60 includes a central processing unit (CPU) 190, a read only memory (ROM) 192, a random access memory (RAM) 194, a storage unit (here, hard disk drive (HDD)) 196, a network interface 197, a display unit 193, an input unit 195, and a portable storage medium drive 199 capable of reading data stored in a portable storage medium 191. The display unit 193 includes a liquid crystal display, and the input unit 195 includes a keyboard, a mouse, and a touch panel. Each of these components of the physical therapist terminal 60 is connected to a bus 198. Furthermore, the physical therapist terminal 60 includes a communication unit that communicates wirelessly or by wire with the sensing device 50.

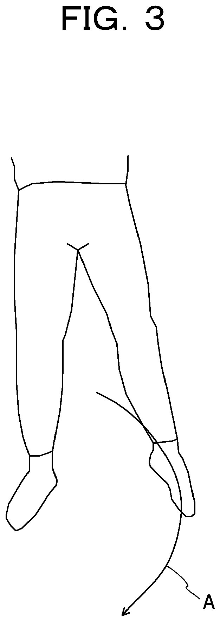

Returning to FIG. 1, the doctor terminal 70 is a terminal used by a doctor, such as a PC. The doctor terminal 70 is a terminal that displays information transmitted from the server 10 and presents the information to a doctor. As illustrated in FIG. 2A, the doctor terminal 70 has a similar hardware configuration to that of the physical therapist terminal 60. A doctor refers to information displayed on the doctor terminal 70 to confirm a state of a circumduction gait of a patient, examine a treatment policy, and confirm an effect of medication. Here, the circumduction gait is a compensatory movement performed by a patient with stroke or the like during walking, and means walking forward while moving a leg so as to throw out the leg in a right and left direction, as illustrated in FIG. 3.

The server 10 calculates a feature amount (output feature amount) related to a circumduction gait of a patient on the basis of data indicating change in an angular velocity over time acquired from the physical therapist terminal 60. Furthermore, the server 10 outputs the calculated output feature amount to the doctor terminal 70.

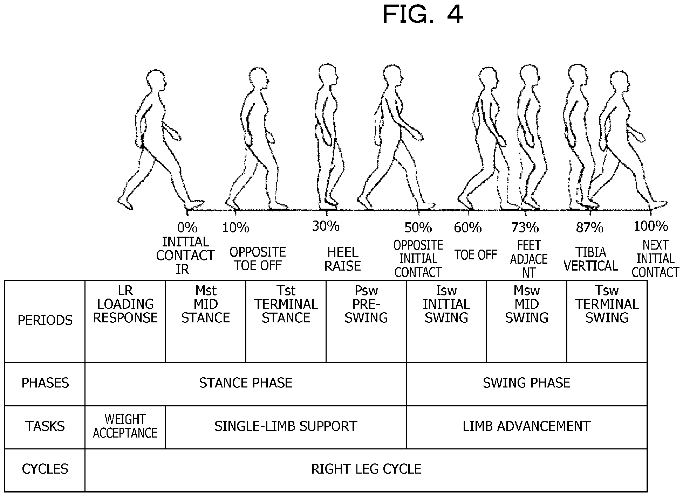

Here, when calculating an output feature amount related to a circumduction gait, the server 10 of the present embodiment calculates an index in accordance with a walking cycle used in a current clinical site. The most used walking cycle in a clinical site is a walking cycle called "Rancho Los Amigos method" as illustrated in FIG. 4, which was developed by Kirsten Götz-Neumann, a German physical therapist at the Rancho Los Amigos National Rehabilitation Center. In the Rancho Los Amigos method, a walking cycle is divided into eight phases. The walking cycle means from when a leg touches the ground until the same leg touches the ground again. Furthermore, the eight phases of the walking cycle are initial contact (IR), loading response (LR), mid stance (Mst), terminal stance (Tst), pre-swing (Psw), initial swing (Isw), mid swing (Msw), and terminal swing (Tsw). Among these, a time from IR to Psw is called a stance phase, and a time from Isw to Tsw is called a swing phase. In the stance phase, a target leg (right leg in FIG. 4) is on a floor (ground), and in the swing phase, the target leg is off the floor (ground). In the present embodiment, the swing phase of the walking cycle is focused on to calculate an output feature amount related to a circumduction gait.

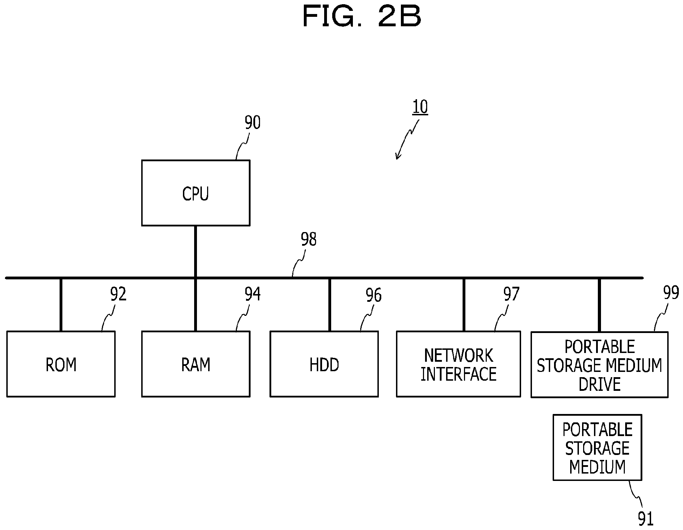

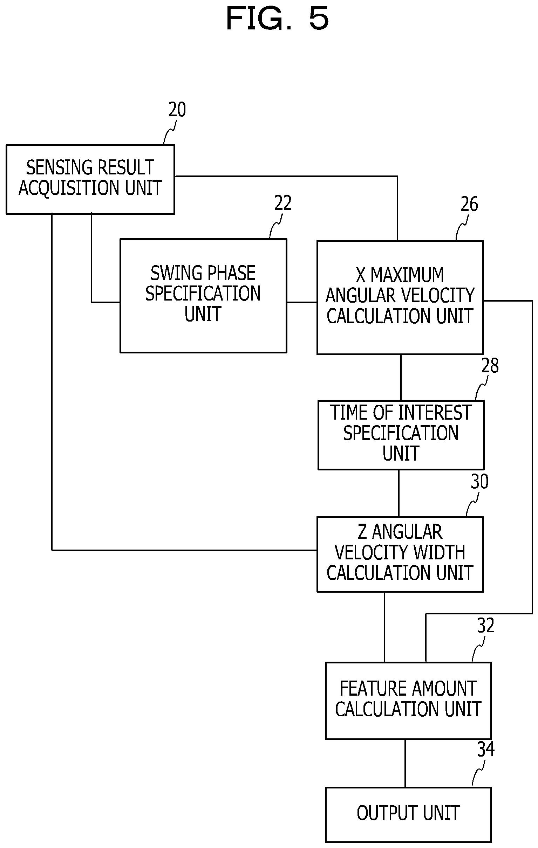

FIG. 2B illustrates a hardware configuration of the server 10. The server 10 includes a CPU 90, a ROM 92, a RAM 94, a storage unit (here, HDD) 96, a network interface 97, and a portable storage medium drive 99. Each of these components of the server 10 is connected to a bus 98. In the server 10, the CPU 90 executes a program (including an information processing program) stored in the ROM 92 or the HDD 96 or a program (including an information processing program) read by the portable storage medium drive 99 from a portable storage medium 91, to implement a function of each unit illustrated in FIG. 5. Note that the function of each unit in FIG. 5 may be implemented by, for example, an integrated circuit such as an application specific integrated circuit (ASIC) or a field programmable gate array (FPGA).

FIG. 5 illustrates a functional block diagram of the server 10. In the server 10, the CPU 90 executes a program to implement functions as a sensing result acquisition unit 20 as an acquisition unit, a swing phase specification unit 22 as a detection unit, an X maximum angular velocity calculation unit 26, a time of interest specification unit 28, a Z angular velocity width calculation unit 30, a feature amount calculation unit 32, and an output unit 34.

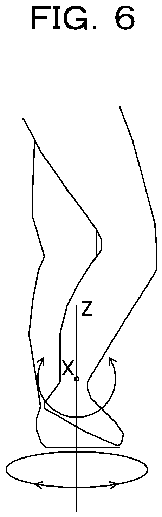

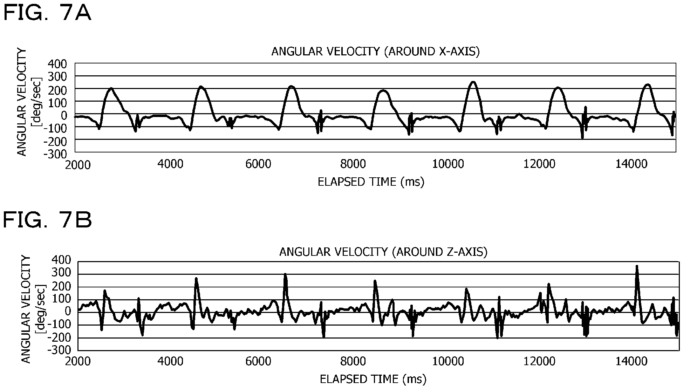

The sensing result acquisition unit 20 acquires, from the physical therapist terminal 60, data of change in an angular velocity over time detected by the sensing device 50. Here, the sensing device 50 detects an angular velocity around a first axis (X-axis) extending in a right and left direction of a patient, and an angular velocity around a second axis (Z-axis) extending in a vertical direction, which are indicated by arrows in FIG. 6. Note that data of change in an angular velocity over time around the X-axis acquired in one walk test is, for example, data as illustrated in FIG. 7A, and data of change in an angular velocity over time around the Z-axis acquired in one walk test is, for example, data as illustrated in FIG. 7B.

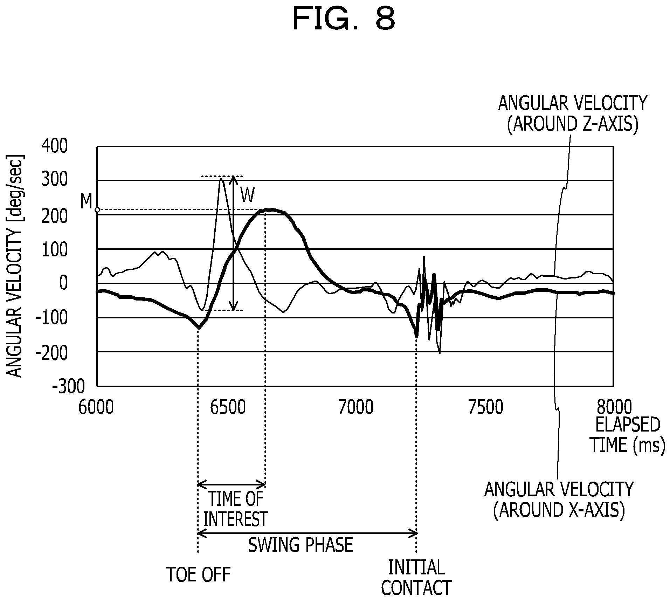

The swing phase specification unit 22 detects a timing when a foot is off the floor (ground) (toe off) on the basis of data of change in an angular velocity over time around the X-axis. The toe off timing is a switching timing between pre-swing (Psw) and initial swing (Isw) in FIG. 4. FIG. 8 extracts part of the data of change in an angular velocity over time illustrated in FIGs. 7A and 7B, which is superimposed and displayed on the same coordinate system. As the toe off timing, the swing phase specification unit 22 detects a timing when a value of an angular velocity around the X-axis indicates a local minimum value immediately before the value increases significantly. Note that the swing phase specification unit 22 detects the toe off timing by, for example, differentiating the data of change in the angular velocity over time around the X-axis.

Furthermore, the swing phase specification unit 22 detects a timing when the foot that is off the floor (ground) touches the floor (ground) (initial contact) on the basis of the data of change in the angular velocity over time. In the example of FIG. 8, as the initial contact timing, the swing phase specification unit 22 detects a timing when the value of the angular velocity around the X-axis indicates a local minimum value after the value increases significantly. Note that the swing phase specification unit 22 detects the initial contact timing by, for example, differentiating the data of change in the angular velocity over time around the X-axis.

Moreover, the swing phase specification unit 22 specifies, as the swing phase, a time between the toe off timing and the initial contact timing.

The X maximum angular velocity calculation unit 26 calculates a timing when an angular velocity around the X-axis reaches a maximum value within a time of the swing phase specified by the swing phase specification unit 22 and the maximum value (refer to M in FIG. 8).

The time of interest specification unit 28 specifies, as a time of interest, a time between a toe off timing detected by the swing phase specification unit 22 and a timing when an angular velocity around the X-axis indicates a maximum value, which is calculated by the X maximum angular velocity calculation unit 26. Note that, in the present embodiment, it can be said that the time of initial swing (Isw) in FIG. 4 is specified as the time of interest.

The Z angular velocity width calculation unit 30 calculates a difference (width) between a minimum value and a maximum value of angular velocities around the Z-axis (refer to W in FIG. 8) with reference to change in an angular velocity around the Z-axis within a time of interest specified by the time of interest specification unit 28. This width W means a degree of a circumduction gait (degree of a swing of a leg in a lateral direction) in the initial swing (Isw).

The feature amount calculation unit 32 calculates a feature amount for determining whether or not a patient is performing a circumduction gait on the basis of a width W calculated by the Z angular velocity width calculation unit 30 and a maximum value M of an angular velocity around the X-axis calculated by the X maximum angular velocity calculation unit 26. Here, the feature amount calculation unit 32 calculates a feature amount C on the basis of the following Equation (1) as an example.

$$C = W/M \ \ldots (1)$$

In the present embodiment, normalization is performed by using a value obtained by dividing the width W by the maximum value M as the feature amount. However, the present invention is not limited to this, and the feature amount may be the width W itself.

Note that, since a plurality of times of interest is specified from the data of one walk test illustrated in FIGs. 7A and 7B, the feature amount calculation unit 32 calculates a plurality of feature amounts corresponding to the times of interest from the data of one walk test.

Return to FIG. 5, the output unit 34 obtains an average value of a plurality of feature amounts calculated by the feature amount calculation unit 32, and uses the obtained value as an output feature amount. Furthermore, the output unit 34 outputs the output feature amount to the doctor terminal 70.

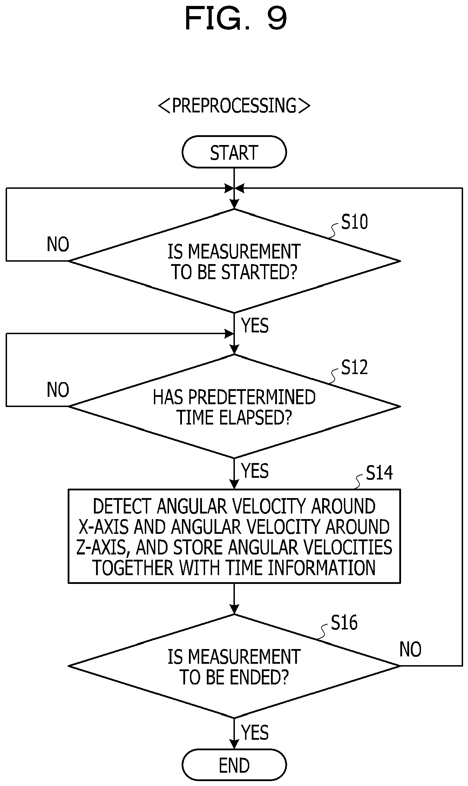

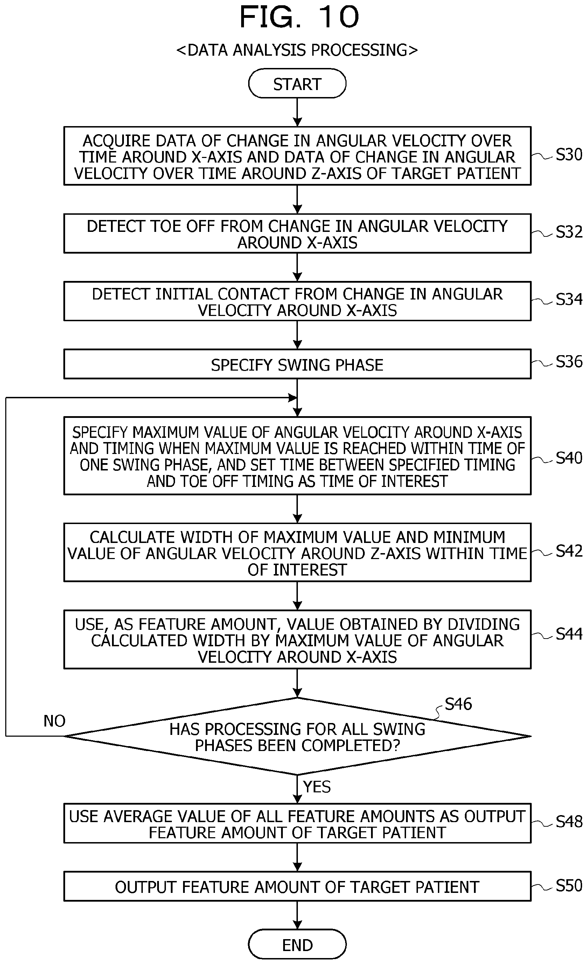

Next, flows of preprocessing by the sensing device 50 and data analysis processing by the server 10 will be described with reference to flowcharts in FIGs. 9 and 10.

(Regarding Preprocessing by Sensing Device 50)

FIG. 9 illustrates a flowchart of the preprocessing by the sensing device 50. In this preprocessing, the sensing device 50 acquires data of change in an angular velocity over time while a patient is performing a walk test under instructions of a physical therapist. As a premise of the processing in FIG. 9, it is assumed that the patient has the sensing devices 50 attached to both ankles and is ready for the walk test. The walk test is performed for a distance of 10 m, for example, and the sensing devices 50 detect angular velocities of both legs while the patient is walking straight for the distance of 10 m.

In the processing of FIG. 9, first, in Step S10, the control unit of the sensing device 50 stands by until an instruction to start measurement is input. For example, in a case where the input button for inputting measurement start provided in the sensing device 50 is pressed by the physical therapist or the like, the control unit advances the processing to Step S12.

When the processing proceeds to Step S12, the control unit stands by until a predetermined time elapses. The predetermined time here means a measurement interval, and is assumed to be about several ms to several tens of ms.

When the predetermined time has elapsed and the processing proceeds to Step S14, the control unit detects an angular velocity around the X-axis and an angular velocity around the Z-axis by using the angular velocity sensor, and stores the angular velocities in the memory together with time information.

Next, in Step S16, the control unit determines whether or not to end the measurement. For example, in a case where the input button for inputting measurement end is pressed by the physical therapist or the like, the determination in Step S16 is positive, but in a case where the input button is not pressed, the processing returns to Step S12. Thereafter, the control unit repeatedly executes the processing and determination in Steps S12 to S16 until the determination in Step S16 becomes positive. Then, when the determination in Step S16 is positive, the entire processing in FIG. 9 is ended.

As described above, by performing the processing of FIG. 9, it is possible to store, in the memory, data of change in the angular velocity over time around the X-axis and data of change in the angular velocity over time around the Z-axis of a leg during the walk test performed by the patient (for example, the data in FIGs. 7A and 7B). Note that the sensing devices 50 are provided on both ankles during the walk test. Thus, after the walk test is completed, the data of FIGs. 7A and 7B will be acquired for both legs.

Note that, the data stored in the memory by the processing of FIG. 9 is transmitted to the physical therapist terminal 60 when the sensing device 50 is connected to the physical therapist terminal 60. It is assumed that the physical therapist transmits the data to the server 10 after associating each piece of data with the patient on the physical therapist terminal 60.

(Regarding Data Analysis Processing by Server 10)

Next, the data analysis processing by the server 10 will be described in detail on the basis of FIG. 10. The processing of FIG. 10 is assumed to be started at a timing when data of change in an angular velocity over time of a patient (target patient) is transmitted from the physical therapist terminal 60, as an example.

In the processing of FIG. 10, first, in Step S30, the sensing result acquisition unit 20 acquires data of change in an angular velocity over time around the X-axis and data of change in an angular velocity over time around the Z-axis of the target patient. Here, it is assumed that the sensing result acquisition unit 20 has acquired the data in FIGs. 7A and 7B as data of one leg of the target patient.

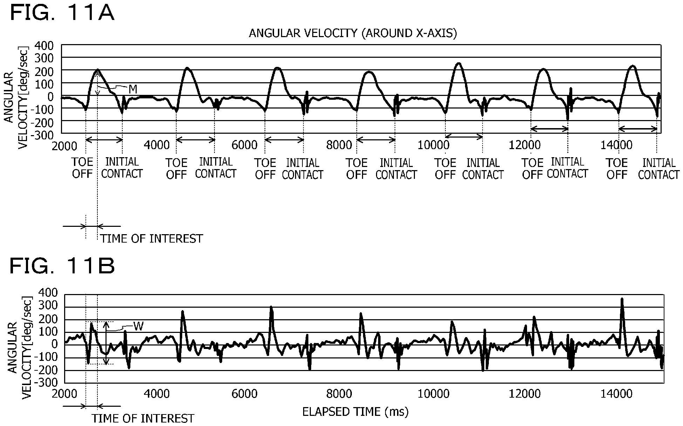

Next, in Step S32, the swing phase specification unit 22 detects toe off from the change in the angular velocity around the X-axis. From the data in FIG. 7A, toe off is detected a plurality of times as illustrated in FIG. 11A.

Next, in Step S34, the swing phase specification unit 22 detects initial contact from the change in the angular velocity around the X-axis. From the data in FIG. 7A, initial contact is detected a plurality of times as illustrated in FIG. 11A.

Next, in Step S36, the swing phase specification unit 22 specifies a swing phase. In this case, the swing phase specification unit 22 specifies each of times between toe off and initial contact as the swing phase, which are indicated by solid double-headed arrows in FIG. 11A.

Note that the swing phase specification unit 22 may detect one toe off and one initial contact in Steps S32 and S34, and specify one swing phase in Step S36. In this case, the swing phase specification unit 22 need only specify a plurality of swing phases by repeating Steps S32 to S36.

Next, in Step S40, the X maximum angular velocity calculation unit 26 selects one swing phase, and specifies a timing when the angular velocity around the X-axis reaches a maximum value M (value indicated by a dashed double-headed arrow in FIG. 11A) and the maximum value M within a time of the selected swing phase. Furthermore, the time of interest specification unit 28 sets a time between the specified timing and a toe off timing as a time of interest. In the example of FIG. 11A, a state where a swing phase at the left end of FIG. 11A is selected and a time of interest is set is indicated.

Next, in Step S42, the Z angular velocity width calculation unit 30 calculates a width of a maximum value and a minimum value of the angular velocity around the Z-axis within the time of interest. In this case, the Z angular velocity width calculation unit 30 calculates a width W illustrated in FIG. 11B.

Next, in Step S44, the feature amount calculation unit 32 uses, as a feature amount, a value obtained by dividing the width W calculated in Step S42 by the maximum value M of the angular velocity around the X-axis specified in Step S40 (refer to the above Equation (1)).

Next, in Step S46, the feature amount calculation unit 32 determines whether or not the processing for all the swing phases has been completed. In a case where the determination in step S46 is negative, the processing returns to step S40, and the processing and determination in Steps S40 to S46 are repeated until the determination in Step S46 becomes positive. Then, when the determination in Step S46 is positive, the output unit 34 executes processing of Step S48.

In Step S48, the output unit 34 calculates an average value of all feature amounts, and uses the average value as an output feature amount of the target patient. For example, in the examples of FIGs. 11A and 11B, since seven feature amounts may be obtained in seven times of interest, the output unit 34 uses an average value of these seven feature amounts as an output feature amount of the target patient.

Next, in Step S50, the output unit 34 outputs the output feature amount of the target patient calculated in Step S48 to the doctor terminal 70. Thus, the entire processing in FIG. 10 ends.

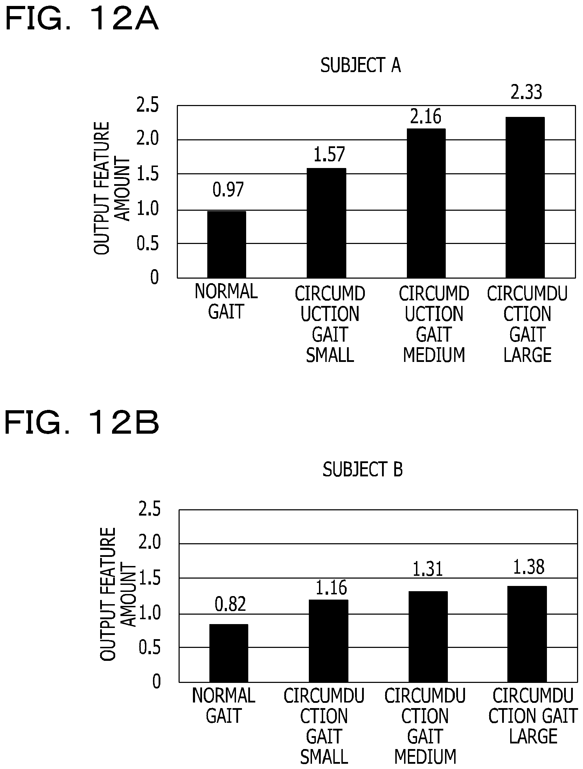

FIGs. 12A and 12B are graphs illustrating output feature amounts output when subjects A and B performed a walk test, with two healthy persons as the subjects A and B. In this walk test, a reference mark for a swing amount in a right and left direction of a leg when a circumduction gait (small, medium, or large) is performed was provided on a floor, and the two subjects A and B having the sensing devices 50 attached to both ankles were asked to perform a normal gait, a circumduction gait (small), a circumduction gait (medium), and a circumduction gait (large). Then, output feature amounts were calculated based on detection results of the sensing devices 50 during the walk test. Note that the output feature amounts in FIGs. 12A and 12B are calculated on the basis of data obtained from the sensing devices 50 attached to the ankles of the swinging legs.

As illustrated in FIGs. 12A and 12B, although there are individual differences in the magnitude of the value of the feature amount, it can be seen that, for the same subject, the value of the output feature amount decreases in the order of the circumduction gait (large), the circumduction gait (medium), the circumduction gait (small), and the normal gait.

Therefore, a doctor may confirm whether a circumduction gait of a patient is improved by observing change in an output feature amount for each patient. This enables the doctor to appropriately perform follow-up in a case where a patient is undergoing rehabilitation and confirm an effect of medicine. Furthermore, since the doctor may appropriately perform follow-up and confirm an effect of medicine, the doctor may appropriately construct rehabilitation plans and medication plans, perform clinical trials, and the like. Furthermore, in a case where change in an output feature amount of a target patient is similar to that of another patient, the doctor may perform construction of rehabilitation plans and medication plans, and the like with reference to data of treatment executed for the another patient.

As can be seen from the description so far, in the present embodiment, the X maximum angular velocity calculation unit 26 and the time of interest specification unit 28 implement a function as a specification unit that specifies a predetermined time from a beginning of a swing phase as a time of interest. Furthermore, in the present embodiment, the Z angular velocity width calculation unit 30, the feature amount calculation unit 32, and the output unit 34 implement a function as a calculation unit that calculates an index (output feature amount) used to determine, on the basis of change in an angular velocity over time around the Z-axis within the time of interest, whether or not a person is performing a circumduction gait.

As described in detail above, according to the present embodiment, the sensing result acquisition unit 20 acquires data of change in an angular velocity over time of an ankle during walking detected by the sensing device 50 attached to the ankle of a patient. Furthermore, the swing phase specification unit 22 specifies a time of a swing phase based on change in an angular velocity over time around an X-axis extending in a right and left direction of the patient among pieces of the data of change in the angular velocity over time. Furthermore, the time of interest specification unit 28 specifies, as a time of interest, a time from a timing at a beginning of the swing phase to a timing when the angular velocity around the X-axis becomes maximum in the swing phase, which is calculated by the X maximum angular velocity calculation unit 26. Then, the feature amount calculation unit 32 calculates an index (feature amount) used to determine whether or not the patient is performing a circumduction gait based on a width W of an angular velocity around a Z-axis extending in a vertical direction within the time of interest, which is calculated by the Z angular velocity width calculation unit 30. Therefore, in the present embodiment, since the feature amount for detecting a circumduction gait is calculated in accordance with a walking cycle used in a clinical site (Rancho Los Amigos method), a doctor may accurately determine (analyze) whether or not the patient is performing a circumduction gait. Furthermore, in the present embodiment, since the sensing device 50 is attached to the ankle of the patient during a walk test, muscles and thick blood vessels are not pressed, unlike in a case where the sensing device 50 is attached to a thigh. Therefore, a burden on the patient may be reduced.

Furthermore, in the present embodiment, since a time from the beginning of the swing phase to the time when the angular velocity around the X-axis becomes maximum within the swing phase is used as the time of interest, the time of initial swing (Isw) may be set as the time of interest.

Furthermore, in the present embodiment, the feature amount is calculated on the basis of the above Equation (1). Thus, in the present embodiment, since a value obtained by dividing the width W of a maximum value and a minimum value of the angular velocity around the Z-axis within the time of interest by a maximum value of the angular velocity around the X-axis in the swing phase is adopted as the feature amount, a value normalized in consideration of a walking speed of the patient may be used as the feature amount. Therefore, it is possible to provide the doctor with an appropriate value as the feature amount.

Note that, in the embodiment described above, the case has been described where the time of interest specification unit 28 specifies the time of initial swing (Isw) as the time of interest. However, the present invention is not limited to this. For example, the time of interest may be the time of the initial swing (Isw) and a time of pre-swing (Psw). Note that the pre-swing (Psw) is generally a predetermined time (for example, 250 ms) before toe off.

Note that, in the embodiment described above, the case has been described where the time of interest specification unit 28 specifies the time of initial swing (Isw) as the time of interest, and uses, as the initial swing, the time between the toe off timing and the timing when the angular velocity around the X-axis indicates a maximum value. However, the present invention is not limited to this, and a time until a predetermined time (for example, 250 ms) elapses after the toe off (beginning of the swing phase) timing may be used as the initial swing (Isw).

Note that, in the embodiment described above, the case has been described where the feature amount is obtained from the above Equation (1). However, the present invention is not limited to this. For example, instead of the difference (width W) between the minimum value and the maximum value of the angular velocity around the Z-axis within the time of interest, the maximum value of the angular velocity around the Z-axis within the time of interest may be used, or an area obtained by time-integrating a graph of the angular velocity around the Z-axis within the time of interest, or the like may be used. Furthermore, instead of the maximum value M of the angular velocity around the X-axis in the swing phase, a width of the maximum value and a minimum value of the angular velocity around the X-axis in the swing phase, an area obtained by time-integrating a graph of the angular velocity around the X-axis in the swing phase, a moving speed relative to a traveling direction of the patient, or the like may be used.

(Modification)

Hereinafter, a modification will be described. In the present modification, a swing amount in a right and left direction (lateral movement amount) of a leg in a case where a patient performs a circumduction gait is used as a feature amount, and a maximum value of the feature amount is used as an output feature amount.

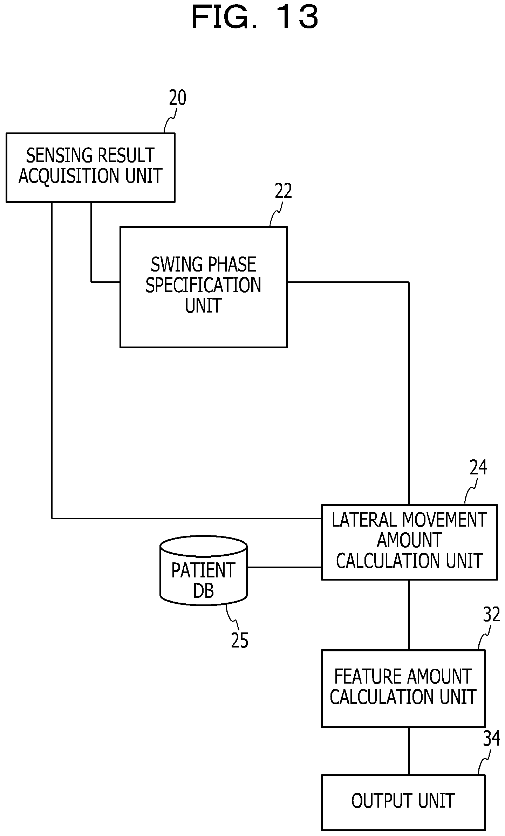

FIG. 13 illustrates a functional block diagram of a server 10 in the present modification. The CPU 90 executes a program in the server 10 to implement functions as illustrated in FIG. 13. Note that, as can be seen by comparing FIGs. 13 and 5, the server 10 of the present modification has a function as a lateral movement amount calculation unit 24 instead of the functions of the X maximum angular velocity calculation unit 26, the time of interest specification unit 28, and the Z angular velocity width calculation unit 30 of the embodiment described above (FIG. 5).

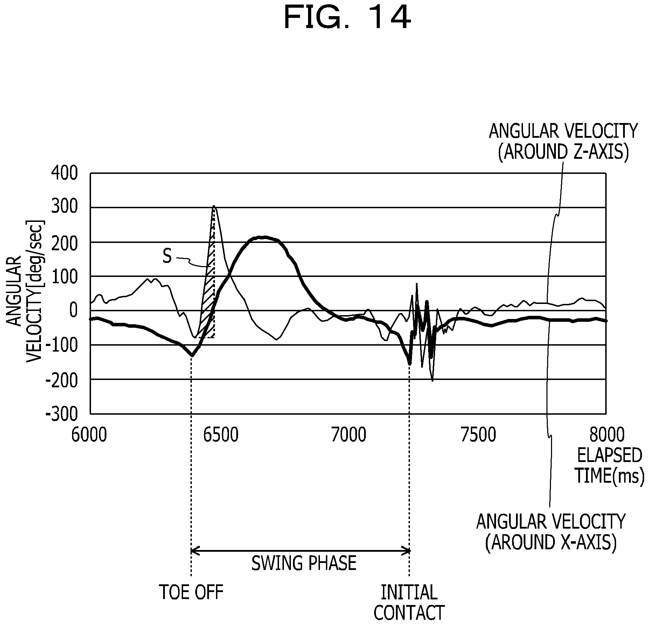

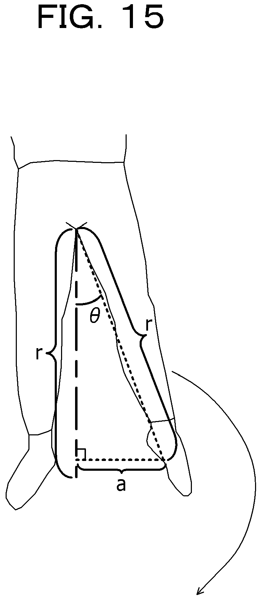

The lateral movement amount calculation unit 24 acquires a swing phase specified by a swing phase specification unit 22 in a similar manner as in the embodiment described above. Then, the lateral movement amount calculation unit 24 uses a time of the swing phase itself as a time of interest to calculate an area S (refer to a hatched part in FIG. 14) obtained by time-integrating a graph from a first local minimum value to the next local maximum value within the time of interest. This area S corresponds to an opening angle $\theta$ in a right and left direction of legs illustrated in FIG. 15.

Thus, the lateral movement amount calculation unit 24 acquires a length r of a leg of a patient from a patient DB 25 (refer to FIG. 13) that stores information regarding the length of the leg of the patient, and calculates, from r and $\theta$, a lateral movement amount a of the leg on the basis of the following Equation (2).

$$a = r \times \sin\theta \ ... \ (2)$$

The lateral movement amount calculation unit 24 transmits the calculated lateral movement amount a to the feature amount calculation unit 32. The feature amount calculation unit 32 uses a plurality of lateral movement amounts a obtained from data of one walk test as feature amounts.

The output unit 34 sets a maximum value of a plurality of feature amounts (lateral movement amounts a) as an output feature amount related to a circumduction gait of a patient. Then, the output unit 34 transmits the output feature amount to the doctor terminal 70.

Note that, in the present modification, the lateral movement amount calculation unit 24, the feature amount calculation unit 32, and the output unit 34 implement a function as a calculation unit that calculates an index (output feature amount) used to determine whether or not a patient is performing a circumduction gait.

As described above, also in the present modification, since the output feature amount related to a circumduction gait is calculated in accordance with a walking cycle used in a clinical site (Rancho Los Amigos method), a doctor may appropriately determine whether or not the patient is performing a circumduction gait. Note that the lateral movement amount a of the leg does not have to be obtained from an angular velocity around a Z-axis as described above. For example, the lateral movement amount a may be calculated on the basis of a detection result of an acceleration sensor or a speed sensor.

Note that, in the modification described above, the case has been described where the feature amount calculation unit 32 calculates the lateral movement amount a of the leg by using the length r and the opening angle θ of the legs of the patient and uses the lateral movement amount a as the feature amount. However, the present invention is not limited to this. For example, the feature amount may be the opening angle θ itself. Also in this way, a doctor may confirm improvement of a symptom, an effect of treatment, and the like by observing change in the feature amount.

Note that, in the embodiment and the modification described above, the case has been described where the processing in FIG. 10 is executed by the server 10. However, the present invention is not limited to this. For example, the physical therapist terminal 60 or the doctor terminal 70 may execute part or all of the processing in FIG. 10. For example, the processing in FIG. 10 need only be implemented by one or a plurality of devices in the information processing system 100.

Note that, in the embodiment and the modification described above, the case has been described where the sensing device 50 is connected to the physical therapist terminal 60, but the present invention is not limited to this, and the sensing device 50 may be connected to the doctor terminal 70. In this case, the doctor terminal 70 need only perform processing similar to the processing performed by the physical therapist terminal 60 of the embodiment described above.

Note that, in the embodiment and the modification described above, the case has been described where the output unit 34 outputs the feature amount to the doctor terminal 70, but the present invention is not limited to this, and the output unit 34 may output the feature amount to other terminals. For example, the output unit 34 may output the feature amount to the physical therapist terminal 60 or a terminal of a pharmaceutical company conducting a clinical trial.

Note that the processing functions described above may be implemented by a computer. In that case, a program is provided in which processing content of a function that a processing device should have is described. The program is executed on the computer, whereby the processing functions described above are implemented on the computer. The program in which the processing content is described may be recorded in a computer-readable storage medium (note that a carrier wave is excluded).

In a case of distributing the program, for example, the program is sold in a form of a portable storage medium such as a digital versatile disc (DVD) or a compact disc read only memory (CD-ROM) in which the program is recorded. Furthermore, it is also possible to store the program in a storage device of a server computer and transfer the program from the server computer to another computer via a network.

The computer that executes the program stores, for example, the program recorded in the portable storage medium or the program transferred from the server computer in a storage device of the computer. Then, the computer reads the program from the storage device of the computer and executes processing according to the program. Note that the computer may also read the program directly from the portable storage medium and execute processing according to the program. Furthermore, the computer may also sequentially execute processing according to the received program each time the program is transferred from the server computer.

The embodiment described above is an example of a preferred embodiment of the present invention. However, the present invention is not limited to this, and various modifications may be made without departing from the scope of the present invention.

[Reference Signs List]

20    Sensing result acquisition unit (Acquisition unit)
22    Swing phase specification unit (Detection unit)
26    X maximum angular velocity calculation unit (Part of specification unit)
28    Time of interest specification unit (Part of specification unit)
30    Z angular velocity width calculation unit (Part of calculation unit)
32    Feature amount calculation unit (Part of calculation unit)
34    Output unit (Part of calculation unit)
50    Sensing device (Sensor)
100   Information processing system (100)

**Claims**

1. An information processing program that causes a computer to execute processing of:

acquiring information regarding an angular velocity of an ankle during walking detected by a sensor attached to the ankle of a person; detecting, among pieces of the acquired information regarding the angular velocity, a swing phase, which is a time when a foot is off a ground, based on change in an angular velocity over time around a first axis that extends in a right and left direction of the person; specifying, as a time of interest, a predetermined time from a beginning of the swing phase; and calculating, among the pieces of the acquired information regarding the angular velocity, an index used to determine whether or not the person is performing a circumduction gait based on change in an angular velocity over time around a second axis that extends in a vertical direction within the time of interest.

2. The information processing program according to claim 1, wherein the time of interest is a time from the beginning of the swing phase to a time when the angular velocity around the first axis becomes maximum within the time of the swing phase.

3. The information processing program according to claim 1 or 2,
   wherein the index is a value based on a difference between a maximum value and a minimum value of the angular velocity around the second axis within the time of interest.

4. The information processing program according to claim 3, wherein the index is a value obtained by dividing the difference between the maximum value and the minimum value of the angular velocity around the second axis within the time of interest by a maximum value of the angular velocity around the first axis in the swing phase.

5. The information processing program according to claim 1, wherein, in the processing of calculating, a movement amount in a right and left direction of a leg of the person is calculated from the change in the angular velocity over time around the second axis within the time of interest and a length of the leg of the person, and the calculated movement amount is used as the index.

6. An information processing method by a computer, the information processing method comprising processing of:

   acquiring information regarding an angular velocity of an ankle during walking detected by a sensor attached to the ankle of a person;
   detecting, among pieces of the acquired information regarding the angular velocity, a swing phase, which is a time when a foot is off a ground, based on change in an angular velocity over time around a first axis that extends in a right and left direction of the person;
   specifying, as a time of interest, a predetermined time from a beginning of the swing phase; and
   calculating, among the pieces of the acquired information regarding the angular velocity, an index used to determine whether or not the person is performing a circumduction gait based on change in an angular velocity over time around a second axis that extends in a vertical direction within the time of interest.

7. An information processing system comprising:

   an acquisition unit that acquires information regarding an angular velocity of an ankle during walking detected by a sensor attached to the ankle of a person;
   a detection unit that detects, among pieces of the acquired information regarding the angular velocity, a swing phase, which is a time when a foot is off a ground, based on change in an angular velocity over time around a first axis that extends in a right and left direction of the person;
   a specification unit that specifies, as a time of interest, a predetermined time from a beginning of the swing phase; and
   a calculation unit that calculates, among the pieces of the acquired information regarding the angular velocity, an index used to determine whether or not the person is performing a circumduction gait based on change in an angular velocity over time around a second axis that extends in a vertical direction within the time of interest.

8. The information processing system according to claim 7, wherein the time of interest is a time from the beginning of the swing phase to a time when the angular velocity around the first axis becomes maximum within the time of the swing phase.

9. The information processing system according to claim 7 or 8, wherein the index is a value based on a difference between a maximum value and a minimum value of the angular velocity around the second axis within the time of interest.

10. The information processing system according to claim 9, wherein the index is a value obtained by dividing the difference between the maximum value and the minimum value of the angular velocity around the second axis within the time of interest by a maximum value of the angular velocity around the first axis in the swing phase.

11. The information processing system according to claim 7, wherein the calculation unit calculates a movement amount in a right and left direction of a leg of the person from the change in the angular velocity over time around the second axis within the time of interest and a length of the leg of the person, and uses the calculated movement amount as the index.

# FIG. 1

# FIG. 2A

60,70

# FIG. 2B

10

90

CPU

98

| 92 | 94 | 96 | 97 | 99 |
|---|---|---|---|---|
| ROM | RAM | HDD | NETWORK INTERFACE | PORTABLE STORAGE MEDIUM DRIVE |

PORTABLE STORAGE MEDIUM

91

# FIG. 3

A

# FIG. 4

| | 0% INITIAL CONTACT IR | 10% OPPOSITE TOE OFF | 30% HEEL RAISE | 50% OPPOSITE INITIAL CONTACT | 60% TOE OFF | 73% FEET ADJACENT | 87% TIBIA VERTICAL | 100% NEXT INITIAL CONTACT |

| PERIODS | LR LOADING RESPONSE | Mst MID STANCE | Tst TERMINAL STANCE | Psw PRE-SWING | Isw INITIAL SWING | Msw MID SWING | Tsw TERMINAL SWING |
|---|---|---|---|---|---|---|---|
| PHASES | STANCE PHASE | | | | SWING PHASE | | |
| TASKS | WEIGHT ACCEPTANCE | SINGLE-LIMB SUPPORT | | | LIMB ADVANCEMENT | | |
| CYCLES | RIGHT LEG CYCLE | | | | | | |

# FIG. 5

20

SENSING RESULT
ACQUISITION UNIT

22

SWING PHASE
SPECIFICATION
UNIT

26

X MAXIMUM
ANGULAR VELOCITY
CALCULATION UNIT

28

TIME OF INTEREST
SPECIFICATION
UNIT

30

Z ANGULAR
VELOCITY WIDTH
CALCULATION UNIT

32

FEATURE AMOUNT
CALCULATION UNIT

34

OUTPUT UNIT

# FIG. 6

FIG. 7A

ANGULAR VELOCITY (AROUND X-AXIS)

FIG. 7B

ANGULAR VELOCITY (AROUND Z-AXIS)

# FIG. 8

# FIG. 9

<PREPROCESSING>

# FIG. 10

<DATA ANALYSIS PROCESSING>

START

ACQUIRE DATA OF CHANGE IN ANGULAR VELOCITY OVER TIME AROUND X-AXIS AND DATA OF CHANGE IN ANGULAR VELOCITY OVER TIME AROUND Z-AXIS OF TARGET PATIENT — S30

DETECT TOE OFF FROM CHANGE IN ANGULAR VELOCITY AROUND X-AXIS — S32

DETECT INITIAL CONTACT FROM CHANGE IN ANGULAR VELOCITY AROUND X-AXIS — S34

SPECIFY SWING PHASE — S36

SPECIFY MAXIMUM VALUE OF ANGULAR VELOCITY AROUND X-AXIS AND TIMING WHEN MAXIMUM VALUE IS REACHED WITHIN TIME OF ONE SWING PHASE, AND SET TIME BETWEEN SPECIFIED TIMING AND TOE OFF TIMING AS TIME OF INTEREST — S40

CALCULATE WIDTH OF MAXIMUM VALUE AND MINIMUM VALUE OF ANGULAR VELOCITY AROUND Z-AXIS WITHIN TIME OF INTEREST — S42

USE, AS FEATURE AMOUNT, VALUE OBTAINED BY DIVIDING CALCULATED WIDTH BY MAXIMUM VALUE OF ANGULAR VELOCITY AROUND X-AXIS — S44

S46 — HAS PROCESSING FOR ALL SWING PHASES BEEN COMPLETED? — NO

YES

USE AVERAGE VALUE OF ALL FEATURE AMOUNTS AS OUTPUT FEATURE AMOUNT OF TARGET PATIENT — S48

OUTPUT FEATURE AMOUNT OF TARGET PATIENT — S50

END

## FIG. 11A

ANGULAR VELOCITY (AROUND X-AXIS)

TIME OF INTEREST

## FIG. 11B

ELAPSED TIME (ms)

TIME OF INTEREST

## FIG. 12A

SUBJECT A

## FIG. 12B

SUBJECT B

# FIG. 13

20

SENSING RESULT
ACQUISITION UNIT

22

SWING PHASE
SPECIFICATION
UNIT

24

LATERAL MOVEMENT
AMOUNT
CALCULATION UNIT

PATIENT
DB

25

32

FEATURE AMOUNT
CALCULATION UNIT

34

OUTPUT UNIT

# FIG. 14

# FIG. 15

## INTERNATIONAL SEARCH REPORT

| International application No. |
|---|
| PCT/JP2019/002212 |

A.  CLASSIFICATION OF SUBJECT MATTER
Int. Cl. A61B5/11(2006.01)i

According to International Patent Classification (IPC) or to both national classification and IPC

B.  FIELDS SEARCHED

Minimum documentation searched (classification system followed by classification symbols)
Int. Cl. A61B5/06-5/22, A61H1/00-5/00, 99/00

Documentation searched other than minimum documentation to the extent that such documents are included in the fields searched
Published examined utility model applications of Japan       1922-1996
Published unexamined utility model applications of Japan     1971-2019
Registered utility model specifications of Japan             1996-2019
Published registered utility model applications of Japan     1994-2019

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| A | JP 2016-112108 A (CASIO COMPUTER CO., LTD.) 23 June 2016, paragraphs [0001]-[0143], fig. 1-22 & US 2016/0166880 A1, paragraphs [0001]-[0339], fig. 1-22 & CN 105688396 A | 1-11 |
| A | JP 2009-50533 A (NATIONAL UNIVERSITY CORPORATION CHIBA UNIVERSITY) 12 March 2009, paragraphs [0001]-[0035], fig. 1-7 (Family: none) | 1-11 |
| A | JP 2015-136582 A (PANASONIC INTELLECTUAL PROPERTY MANAGEMENT CO., LTD.) 30 July 2015, paragraphs [0001]-[0211], fig. 1-14 (Family: none) | 1-11 |
| A | WO 2017/199305 A1 (FUJITSU LTD.) 23 November 2017, paragraphs [0001]-[0272], fig. 1-73 (Family: none) | 1-11 |

☒ Further documents are listed in the continuation of Box C.　　　☐ See patent family annex.

| | |
|---|---|
| *    Special categories of cited documents: | "T"    later document published after the international filing date or priority date and not in conflict with the application but cited to understand the principle or theory underlying the invention |
| "A"  document defining the general state of the art which is not considered to be of particular relevance | |
| "E"  earlier application or patent but published on or after the international filing date | "X"    document of particular relevance; the claimed invention cannot be considered novel or cannot be considered to involve an inventive step when the document is taken alone |
| "L"  document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"    document member of the same patent family |

| Date of the actual completion of the international search | Date of mailing of the international search report |
|---|---|
| 27.03.2019 | 09.04.2019 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

| International application No. |
| --- |
| PCT/JP2019/002212 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2006-102156 A (THE CIRCLE FOR THE PROMOTION OF SCIENCE AND ENGINEERING) 20 April 2006, paragraphs [0001]-[0116], fig. 1-15 & US 2011/01166488 A1, paragraphs [0001]-[0163], fig. 1-15 & WO 2006/038712 A1 | 1-11 |

Form PCT/ISA/210 (continuation of second sheet) (January 2015)

**EP 3 915 474 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004195109 A

- JP 2016043092 A